# EUROPÄISCHE PATENTSCHRIFT

(11) **EP 2 792 319 B1**
(45) Veröffentlichungstag und Bekanntmachung des Hinweises auf die Patenterteilung: **21.10.2020**
(21) Anmeldenummer: 14000764.2
(22) Anmeldetag: 04.03.2014
(51) Int. Cl.: A61B 17/29

(54) **Betätigungselement und Wippenpaar für ein Betätigungselement**
Actuating element and rocker pair for an actuating element
Élément d'actionnement et paire de bascules pour un élément d'actionnement

(30) Priorität: 17.04.2013 EP 13002016
(43) Veröffentlichungstag der Anmeldung: 22.10.2014
(73) Patentinhaber: JakuTec Medizintechnik GmbH & Co. KG, 78576 Liptingen (DE)
(72) Erfinder: Jakoubek, Franz, 78576 Liptingen (DE)
(74) Vertreter: Glawe, Delfs, Moll

(56) Entgegenhaltungen:
- DE-B4- 10 293 222
- US-A- 5 618 306
- US-A1- 2010 004 677
- US-B1- 6 322 578

## Beschreibung

### Technisches Gebiet

Die vorliegende Erfindung betrifft ein Betätigungselement sowie ein Wippenpaar für ein Betätigungselement. Insbesondere betrifft die vorliegende Erfindung ein Betätigungselement zum Betätigen eines mit dem Griff in Wirkverbindung stehenden Instruments für minimalinvasive Eingriffe.

### Beschreibung des Standes der Technik

Betätigungselemente der gattungsgemäßen Art sind bekannt. So offenbart bspw. die US 2010/0004677 A1 ein zwei relativ zueinander bewegbare Griffhalbschalen umfassendes Betätigungselement, wobei die beiden Griffhalbschalen einenends mittels eines Vorspannmechanismus miteinander verbunden sind und im wesentlichen mittig zwischen den beiden Griffhalbschalen ein Rohr vorgesehen ist. Zur Übertragung einer Betätigungsbewegung der Griffhalbschalen ist ein zwischen den Griffhalbschalen und dem Rohr angeordneter Gelenkmechanismus vorgesehen ist, der mit einer Inneren des Rohrs geführten Stange in Wirkverbindung steht. Eine vergleichbare Ausgestaltung ist auch aus der DE 102 93 222 B4 bekannt. Des weiteren ist aus der US 6,322,578 B1 ein Betätigungselement mit zwei relativ zueinander bewegbaren Griffhalbschalen bekannt, das zwei miteinander in Rastverbindung tretbare Wippen aufweist. Jeweils eine Wippe ist in jeder Griffhalbschale derart angeordnet, dass sie bei einem Schließen der Griffhalbschalen mit entsprechend komplementär ausgebildeten Rastelementen aufeinander treffen. Beim Aufeinandertreffen der Rastelemente drücken sich die beiden Wippen gegenseitig um eine im wesentlichen parallel zur zentralen Rohrachse weg, woraus eine Wippenschwenkbewegung quer zur Erstreckung der Griffhalbschalen resultiert.

Die DE 102 93 222 B4 offenbart ein zangenartiges chirurgisches Element, das zwei relativ zueinander bewegte Griffhalbschalen mit einem mittig dazwischen geführten Rohr und einem zur Übertragung einer Betätigungsbewegung vorgesehenen Gelenkmechanismus sowie einem Rastmechanismus aufweist. Der bekannte Rastmechanismus umfasst zwei Sperrelemente, die an Gelenkstiften gelagert sind, die eine Schwenkbewegung dieser Sperrelemente in einer durch die Griffhalbschalen aufgespannten Ebene gestatten.

### Zusammenfassung der Erfindung

Erfindungsgemäß werden demgegenüber ein Betätigungselement mit den Merkmalen der Patentansprüche 1, 3 bzw. 4 sowie ein Wippenpaar mit den Merkmalen der Patentansprüche 8, 9 bzw. 11 vorgeschlagen.

Demnach weist das Betätigungselement erfindungsgemäß einen Rastmechanismus auf, der es gestattet, das Betätigungselement bei zusammengeführten bzw. zusammengedrückten Griffhalbschalen zu arretieren.

Der Rastmechanismus umfasst eine an einer ersten Griffhalbschale angeordnete Einrastwippe und eine an einer zweiten Griffhalbschale angeordnete Sperrwippe. Bei zusammengeführten Griffhalbschalen treten diese beiden Wippen durch einen dafür vorgesehenen Durchbruch in dem Rohr in eine Sperrrastverbindung.

Eine mögliche Ausgestaltung sieht vor, dass die beiden Wippen durch abermaliges Betätigen der zusammengeführten Griffhalbschalen aus der Sperrrastverbindung lösbar sind.

Eine weitere mögliche Ausgestaltung sieht vor, dass die Sperrwippe ein Rastelement aufweist und die Einrastwippe einen mit dem Rastelement der Sperrwippe in Wirkverbindung bringbaren Hinterschnitt aufweist.

Die Sperrwippe und/oder die Einrastwippe können jeweils eine Stellschraube bzw. Justierschraube umfassen.

Weitere Vorteile und Ausgestaltungen der Erfindung ergeben sich aus der Beschreibung und der beiliegenden Zeichnung.

Es versteht sich, dass die voranstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung ist anhand eines Ausführungsbeispieles in der Zeichnung schematisch dargestellt und wird im folgenden unter Bezugnahme auf die Zeichnung ausführlich beschrieben.

### Kurzbeschreibung der Zeichnung

Figur 1 zeigt in seitlicher Teilschnittdarstellung ein erstes Ausführungsbeispiel eines erfindungsgemäßen Betätigungselements.
Figur 2A zeigt eine perspektivische Darstellung einer erfindungsgemäßen Sperrwippe.
Figur 2B zeigt die Sperrwippe der Figur 2A in seitlicher Draufsicht.
Figur 3A zeigt eine perspektivische Darstellung einer erfindungsgemäßen Einrastwippe.
Figur 3B zeigt die Einrastwippe der Figur 3A in seitlicher Draufsicht.
Figur 4A zeigt die Einrastwippen der Figuren 2A und 3A perspektivisch in Einrastposition.
Figur 4B die Einrastposition der Figur 4A in seitlicher Draufsicht.
Figur 5A zeigt eine perspektivische Darstellung einer erfindungsgemäßen Sperrwippe mit Stellschraube.
Figur 5B zeigt eine perspektivische Darstellung einer erfindungsgemäßen Einrastwippe mit Stellschraube.
Figur 6 zeigt in seitlicher Teilschnittdarstellung ein weiteres Ausführungsbeispiel eines erfindungsgemäßen Betätigungselements mit den Einrastwippen der Figuren 5a und 5B.
Figur 7A zeigt die Einrastwippen der Figuren 5A und 5B perspektivisch in Einrastposition.
Figur 7B die Einrastposition der Figur 7A in seitlicher Draufsicht.
Figur 8 zeigt in seitlicher Teilschnittdarstellung ein drittes Ausführungsbeispiel eines erfindungsgemäßen Betätigungselements.
Figur 9 zeigt in seitlicher Teilschnittdarstellung ein viertes Ausführungsbeispiel eines erfindungsgemäßen Betätigungselements.
Figuren 10A bis 10D zeigen ein weiteres Ausführungsbeispiel eines erfindungsgemäßen Wippenpaares, ähnlich den in den Figuren 5A und 5B gezeigten Wippen.
Figuren 11A bis 11D zeigen ein weiteres Ausführungsbeispiel eines erfindungsgemäßen Wippenpaares, wie es in dem Betätigungselement der Figur 8 eingesetzt ist.

### Ausführliche Beschreibung

In den Figuren sind gleiche Elemente und auch Elemente mit gleicher Funktion mit gleichen Bezugszeichen bezeichnet.

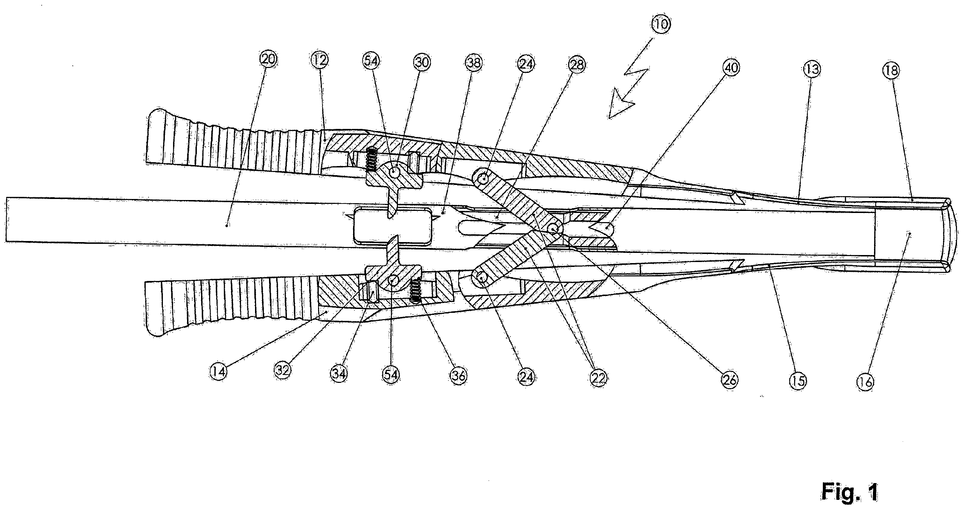

Figur 1 zeigt ein erfindungsgemäßes Betätigungselement 10 zum Betätigen eines mit dem Griff in Wirkverbindung stehenden Instruments für minimalinvasive Eingriffe in seitlicher Teilschnittdarstellung. Zur Vereinfachung der Darstellung und besseren Übersichtlichkeit ist lediglich das Betätigungselement selbst dargestellt. Auf eine Darstellung des Instruments für minimalinvasive Eingriffe (bei dem es sich beispielsweise um eine Klemme, eine Pinzette, eine Schere o.dgl. handeln kann) wurde verzichtet. Die Verbindung des Instruments mit dem Betätigungselement erfolgt in für den Fachmann an sich bekannter Art und Weise.

Das Betätigungselement 10 gemäß der Erfindung umfasst zwei Griffhalbschalen 12, 14, die einenends (13, 15) mittels eines Vorspannmechanismus miteinander verbunden sind. In dem dargestellten Ausführungsbeispiel umfasst der Vorspannmechanismus eine Schaftaufnahme 16 mit einer Grifffeder 18. Die Griffhalbschalen können einstückig mit der Schaftaufnahme bzw. dem Vorspannmechanismus ausgebildet sein. Diese Maßnahme erleichtert die Fertigung und verringert die Reibung zwischen den Teilen während des Betriebs und reduziert den mechanischen Verschleiß. Auch die Grifffeder kann ggf. einstückig mit dem Vorspannmechanismus ausgeführt sein.

Im wesentlichen mittig zwischen den beiden Griffhalbschalen 12, 14 ist ein Rohr 20 geführt, das in an sich bekannter Art und Weise zur Übertragung einer Betätigungsbewegung der Griffhalbschalen 12, 14 dient. Für diese Übertragung einer Relativbewegung der Griffhalbschalen 12, 14 ist ein Gelenkmechanismus vorgesehen, der ein Paar Gelenkstifte 22 umfasst, die mit einem Ende jeweils mittels eines Niets 24 an einer der beiden Griffhalbschalen 12, 14 und andernends an einem gemeinsamen Anlenkpunkt (bzw. Anlenkachse) um einen Stift 26 an einem in dem Rohr 20 geführten Schieber 28 angelenkt sind. Hierfür weist das Rohr 20 einen Axialschlitz 40 auf.

Des weiteren weist das Betätigungselement 10 erfindungsgemäß einen Rastmechanismus auf, der es gestattet, das Betätigungselement 10 bei zusammengedrückten Griffhalbschalen 12, 14 zu arretieren.

Der Rastmechanismus gemäß der Erfindung umfasst eine an einer ersten Griffhalbschale 12 angeordnete Einrastwippe 30 sowie eine an einer zweiten Griffhalbschale 14 angeordnete Sperrwippe 32. Die beiden Wippen 30, 32 kommen bei zusammengeführten Griffhalbschalen 12, 14 durch einen hierfür vorgesehenen Durchbruch 38 des Rohrs 20 miteinander in Kontakt. Die beiden Wippen 30, 32 sind so ausgestaltet, dass sie bei Kontaktaufnahme miteinander verrasten und so eine Rückkehr der vorgespannten Griffhalbschalen 12, 14 verhindern, so dass eine Arretierung der Griffhalbschalen 12, 14 in dieser Position erfolgt.

Bei erneutem Druck auf die Griffhalbschalen 12, 14 löst sich die Verrastung der beiden Wippen 30, 32 wieder, so dass die Griffhalbschalen 12, 14 vorspannungsbedingt in ihre (in der Figur dargestellten) Ausgangslage zurückkehren können.

Die beiden Wippen 30, 32 können bspw. so ausgestaltet sein, dass eine der Wippen 30 einen Hinterschnitt aufweist, der bei Berührung der anderen Wippe 32 mit einem an dieser vorgesehenen Rastelement in Rasteingriff gelangt. Das Rastelement kann bspw. eine Rastnase sein.

Die beiden Wippen 30, 32 sind wie aus der Figur ersichtlich an einer jeweils zugeordneten Griffhalbschale geeignet angelenkt. Die beiden Wippen 30, 32 sind hierzu jeweils um eine Schwenkachse 54 schwenkbar angeordnet. Wie ohne weiteres aus der Figur 1 ersichtlich ist, verläuft die Schwenkachse 54 senkrecht zur Zeichnungsebene und somit im wesentlichen senkrecht zu einer Längsachse des Rohrs 20. Daraus resultiert eine Schwenkbewegung der beiden Wippen 30, 32 in einer Ebene, die durch die beiden Griffhalbschalen 12, 14 aufgespannt ist.

Zur Vorspannung der beiden Wippen sind jeweils eine Druckfeder 36 sowie ein Gewindestift 34 zur Justierung vorgesehen. Die Druckfeder 36 und der Gewindestift 34 sind beiderseits der Wippenanlenkachse angeordnet. Diese aus der Figur gut erkennbare Anordnung der Feder 36 außerhalb des Wippenkörpers gestattet eine sensiblere, d.h. genauere Einstellung der Wippenvorspannung und somit der Wippensperrkraft. Die Feinabstimmung wird dadurch verbessert. Die Verwendung einer (kostengünstigen) Druckfeder anstelle einer Torsionsfeder unterstützt dies, da sie den Druck konstanter abgibt als die Torsionsfeder und somit eine gefühlvollere Betätigung möglich wird.

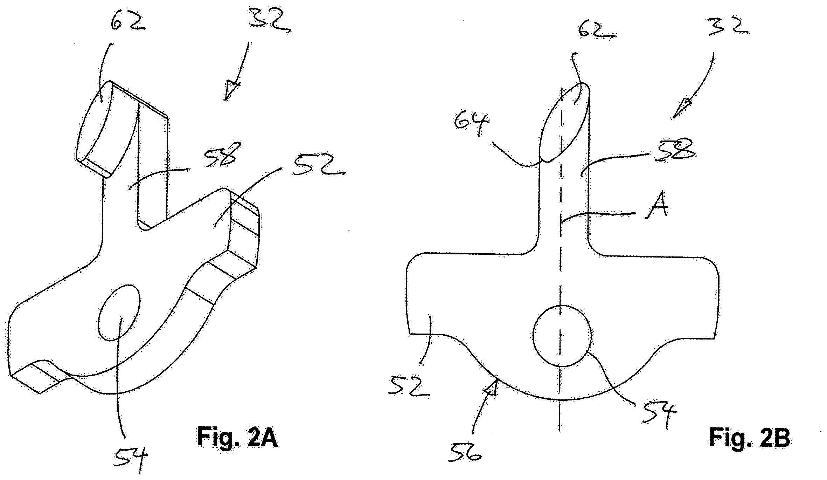

In den Figuren 2 bis 4 sind die beiden erfindungsgemäßen Wippen 30, 32 ausführlicher dargestellt.

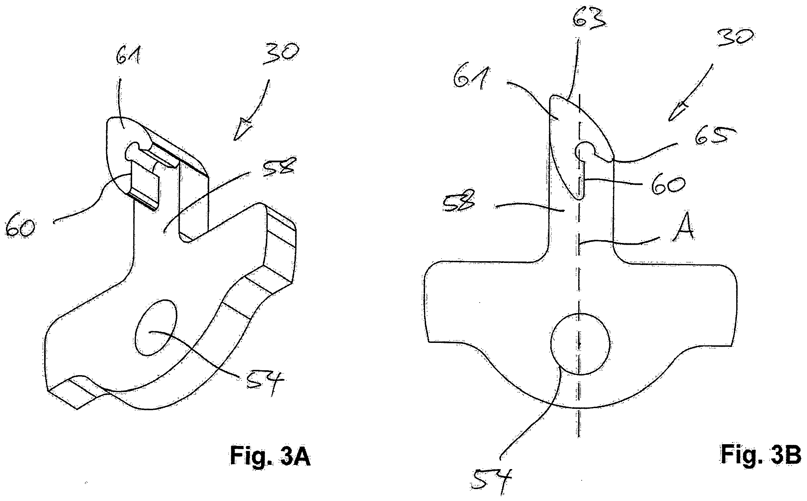

Figur 2A zeigt eine erfindungsgemäße Sperrwippe 32 in perspektivischer Darstellung und Figur 2B in seitlicher Draufsicht. Entsprechend zeigt Figur 3A eine erfindungsgemäße Einrastwippe 30 in perspektivischer Darstellung und Figur 3B in seitlicher Draufsicht.

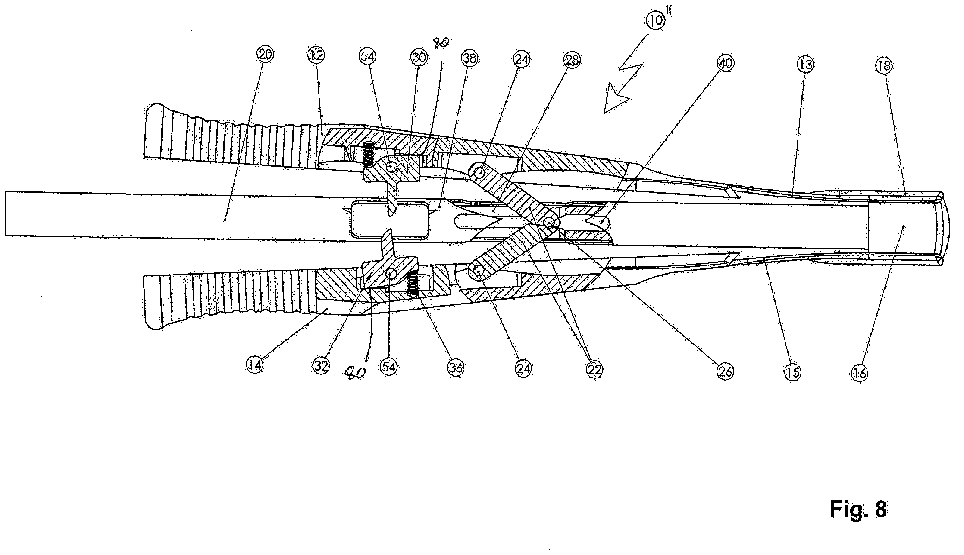

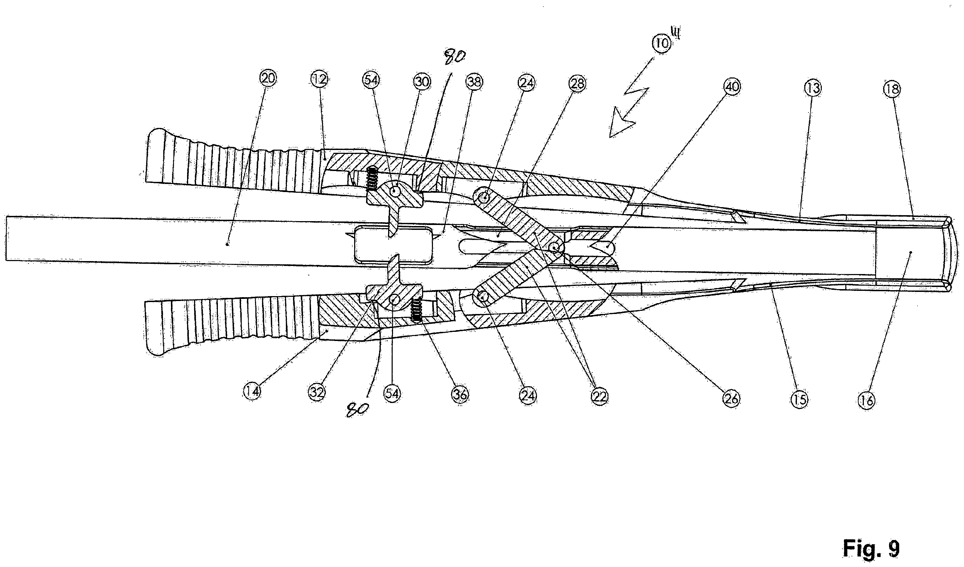

Beide Wippen 30, 32 umfassen jeweils einen Wippenkörper 50 bzw. 52 mit einer zentralen Bohrung zur Durchführung der Schwenkachse 54. Die beiden Wippenkörper 50, 52 sind im wesentlichen gleich gestaltet und weisen insbesondere jeweils eine nockenartig geformte Seite 56 auf. Die Nockenform 56 erleichtert die Führung der Wippe. Beiderseits der Nockenkurve sind Schultern ausgebildet. Eine dieser beiden Schultern dient zur Beaufschlagung mit der Druckfeder 36 (vgl. Figur 1 sowie die Ausführungsbeispiele der Figuren 8 und 9). Die gegenüberliegende Schulter dient im Falle des Ausführungsbeispieles der Figur 1 zur Beaufschlagung mit dem Gewindestift 34.

Auf der der nockenartig geformten Seite 56 gegenüberliegenden Seite verfügt jede Wippe 50, 52 über einen Wippenhals 58. An dem wippenfernen Ende jedes Wippenhalses 58 ist das Rastwirkelement der jeweiligen Wippe ausgeformt.

Die Sperrwippe 32 weist an ihrem Wippenhals 58 ein Rastelement 62 auf. Das Rastelement 62 ragt von dem Wippenhals 58 senkrecht zu dessen Erstreckungsachse A ab. Das Rastelement 62 weist eine im wesentlichen ovale Form auf, die mit ihrer Hauptachse zu der Erstreckungsachse geneigt ist. Die Neigung kann bspw. 15° oder 30° oder 45° betragen (in der Darstellung der Figuren im Uhrzeigersinn gemessen). Andere Neigungswinkel sind ebenfalls möglich und erschließen sich dem Fachmann aus seinem Verständnis der vorliegenden Offenbarung.

Die Einrastwippe 30 weist an ihrem Wippenhals 58 ein Element 61 mit einem Hinterschnitt 60 auf. Das Element 61 ragt von dem Wippenhals 58 senkrecht zu dessen Erstreckungsachse A ab. Das Element 61 weist eine im wesentlichen ovale Form auf, die mit ihrer Hauptachse zu der Erstreckungsachse geneigt ist. Die Neigung ist korrelierend zu der Neigung des Rastelements 62 der Sperrwippe 32 gewählt und kann bspw. 15° oder 30° oder 45° betragen (in der Darstellung der Figuren gegen den Uhrzeigersinn gemessen).

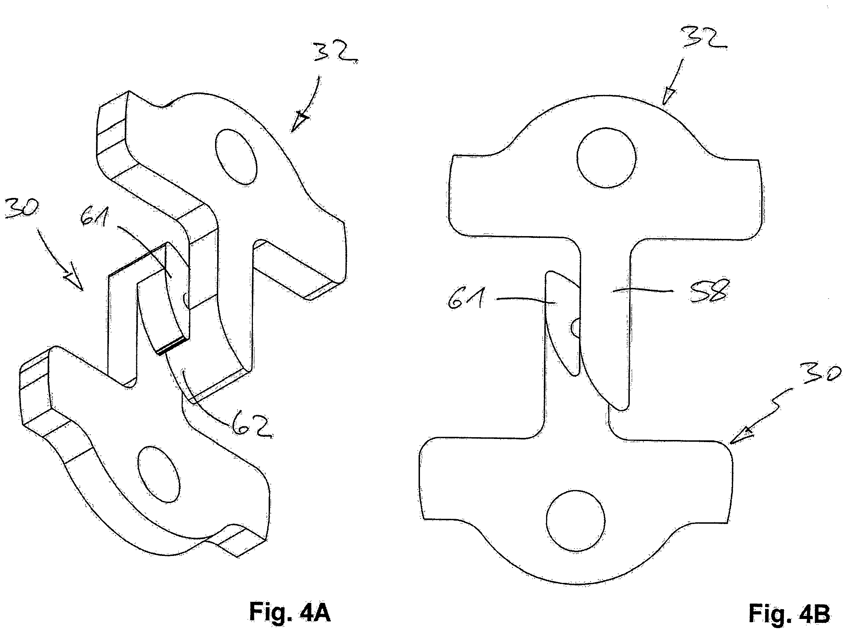

Der Hinterschnitt 60 ist derart an der ovalen Form des Elements 61 angebracht, dass er in Richtung Wippenkörper 56 zeigt und dazu geeignet ist, eine zum Wippenkörper 56 der Sperrwippe 32 weisende Spitze 64 des ovalen Rastelements 62 aufzunehmen. Bei einer Annäherung der beiden gegenüberliegend schwenkbar angeordneten Wippen 30, 32 beaufschlagt die besagte Spitze 64 des Rastelements 32 das Element 61 der Einrastwippe 30 an einer wippenfernen ovalen Fläche 63. Die beiden Wippen drücken sich daraufhin gegenseitig um ihre jeweilige Schwenkachse 54 so zur Seite, dass die Spitze 64 entlang der Fläche 63 gleitet und dann um eine Kante 65 des Hinterschnitts 60 herum in den Hinterschnitt 60 rutscht und einrastet. Diese Verrastungsstellung oder Verrastungsposition ist exemplarisch in den Figuren 4A und 4B dargestellt.

Wie bereits voranstehend beschrieben, löst sich die Verrastung der beiden Wippen 30, 32 bei erneutem Druck auf die Griffhalbschalen 12, 14 wieder, so dass die Griffhalbschalen 12, 14 vorspannungsbedingt in ihre Ausgangslage zurückkehren. Die beschriebene Verschwenkung der beiden Wippen in der durch die beiden Griffhalbschalen aufgespannten Ebene, wie sie den Figuren unschwer entnehmbar ist, hat den Vorteil, dass keine Querbewegung der Wippen quer zu dieser Ebene, wie sie aus dem Stand der Technik bekannt ist, erfolgt, wodurch entsprechende Querkräfte, die im Betrieb des Betätigungselement störend auftreten können, vermieden werden. Das Betätigungselement lässt sich dadurch präziser handhaben, da auf den Griff keine seitliche Druckeinwirkung mehr vorliegt.

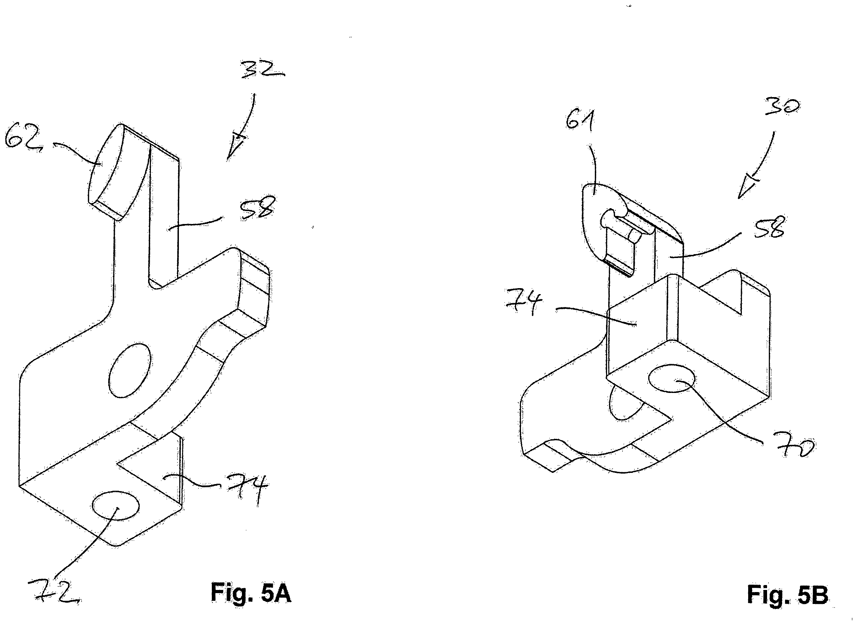

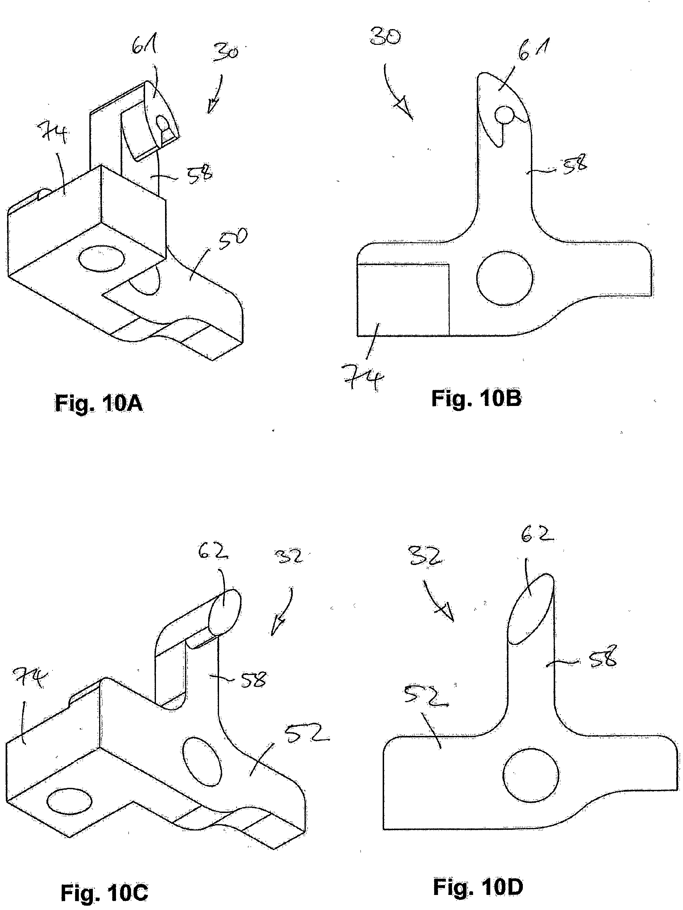

Die Figuren 5A und 5B zeigen Ausgestaltungen der beiden Wippen 30, 32, die zur Aufnahme von (nicht dargestellten) Stellschrauben geeignet ausgebildete Ansätze 74 mit jeweils einer Gewindebohrung 70, 72 aufweisen. Die Gewindebohrung 70, 72 verläuft im wesentlichen parallel zu der Erstreckungsachse A jeder Wippe 30, 32 und im wesentlichen senkrecht zu der Schwenkachse jeder Wippe 30, 32. Mittels der Stellschrauben ist es möglich, die Positionierung jeder Wippe so einzustellen, dass das Rastelement 62 mit seiner Spitze 64 an der gewünschten Stelle auf das Element 61 der Einrastwippe trifft und die beiden Wippen in der Verrastungsstellung richtig stehen, so dass das anschließende Entrasten ohne Komplikationen auf Anhieb und ohne Hakein funktioniert. Ähnliche erfindungsgemäße Wippen sind in den Figuren 10A bis 10D dargestellt, deren Wippenkörper 50, 52 schlanker bzw. schmaler ausgestaltet sind.

Wie den Figuren 3A, 3B sowie 5B unschwer entnehmbar ist, weist der Hinterschnitt 60 des ovalen Elements 61 in seinem innenliegenden spitzen Ende eine Bohrung auf. Die Bohrung kann - wie im Ausführungsbeispiel dargestellt - im wesentlichen kreisrund sein. Die Bohrungsachse verläuft im wesentlichen parallel zu der Schwenkachse 54. Die Bohrung ist derart ausgeführt, dass sie das Ende des darin eingreifenden Elements 62 aufnimmt. Diese Bohrung stellt sicher, dass sich die beiden ineinandergreifenden Elemente 61, 62 der Wippen 30, 32 im eingerasteten Zustand nicht selbsttätig, sprich ohne Betätigung der Wippen über die Griffhalbschalen, voneinander lösen. Oder mit anderen Worten: die Bohrung minimiert das Risiko eines unbeabsichtigten Lösens der Wippensperre.

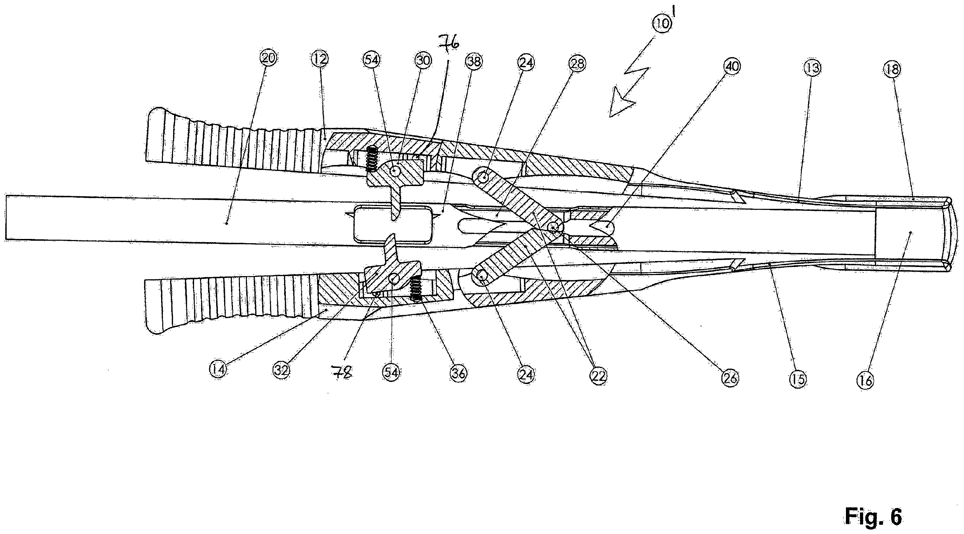

Figur 6 zeigt analog zur Figur 1 in Ausgestaltung ein erfindungsgemäßes Betätigungselement 10' zum Betätigen eines mit dem Griff in Wirkverbindung stehenden Instruments für minimalinvasive Eingriffe in seitlicher Teilschnittdarstellung mit Einrastwippen gemäß der Figuren 5A und 5B und in diesen Einrastwippen eingebrachten Stellschrauben 76 bzw. 78. Die Position jeder Wippe 30, 32 kann durch das Einschraubmaß der betreffenden Stellschraube 76 bzw. 78 in den Gewindebohrungen 70 bzw. 72 der Ansätze 74 der Einrastwippe 30 bzw. der Sperrwippe 32 festgelegt werden.

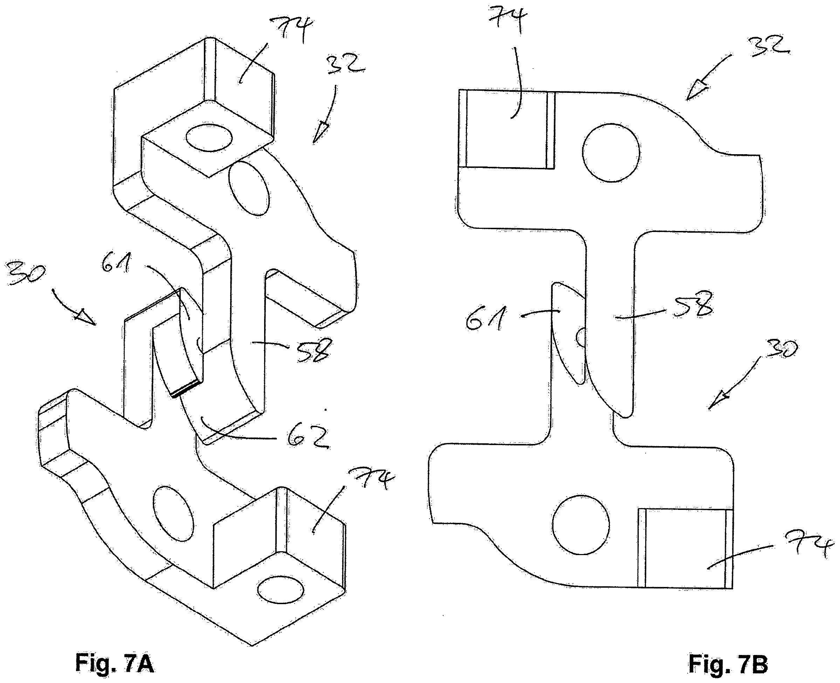

Die Figuren 7A und 7B veranschaulichen entsprechend der Darstellung der Figuren 4A und 4B die Verrastungsposition der Einrastwippen der Figuren 5A und 5B.

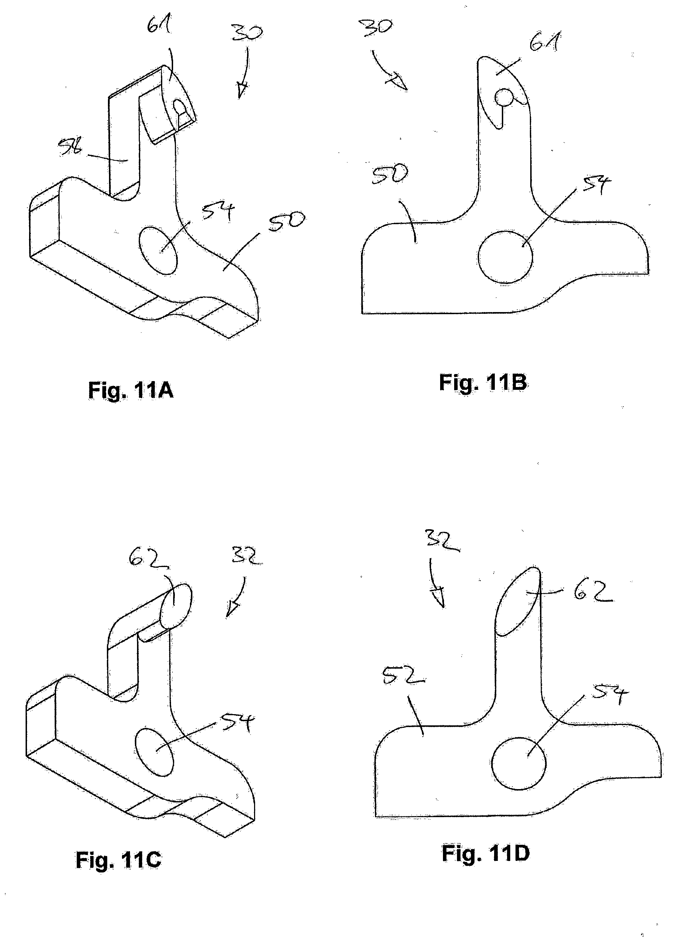

Die Figuren 8 und 9 zeigen zwei weitere Ausführungsformen von erfindungsgemäßen Betätigungselementen 10" bzw. 10''' zum Betätigen eines mit dem Griff in Wirkverbindung stehenden Instruments für minimalinvasive Eingriffe in seitlicher Teilschnittdarstellung. In diesen beiden Ausführungsformen liegt die der Federvorspannung 36 jeweils gegenüberliegende Kante einer Einrastwippe 30, 32 auf einer an der zugeordneten Griffhalbschale ausgebildeten Schulter auf. Die Höhe der Schulter ist dabei auf die Ausgestaltung der Wippenform abgestimmt, so dass die gewünschte Ausrichtung jeder Einrastwippe erzielt wird. Das in dem Betätigungselement 10" der Figur 8 verwendete Wippenpaar 30, 32 ist in den Figuren 11A bis 11D genauer und vergrößert dargestellt.

## Patentansprüche

1. Betätigungselement (10) zum Betätigen eines mit dem Griff in Wirkverbindung stehenden Instruments für minimalinvasive Eingriffe, mit
- zwei relativ zueinander bewegbaren Griffhalbschalen (12, 14), die einenends mittels eines Vorspannmechanismus (16, 18) miteinander verbunden sind, und
- einem im wesentlichen mittig zwischen den beiden Griffhalbschalen (12, 14) geführten Rohr (20), wobei zur Übertragung einer Betätigungsbewegung der Griffhalbschalen (12, 14) ein zwischen den Griffhalbschalen (12, 14) und dem Rohr (20) angeordneter Gelenkmechanismus (22, 24, 26) vorgesehen ist,
wobei das Betätigungselement (10) des weiteren einen Rastmechanismus (30, 32) aufweist, der eine an einer ersten Griffhalbschale (12) angeordnete Einrastwippe (30) und eine an einer zweiten Griffhalbschale (14) angeordnete Sperrwippe (32) umfasst, wobei die beiden Wippen (30, 32) bei zusammengeführten Griffhalbschalen (12, 14) durch einen dafür vorgesehenen Durchbruch in dem Rohr (20) in Sperrrastverbindung treten, und wobei die beiden Wippen (30, 32) derart um jeweils eine senkrecht zu einer Längsachse des Rohrs verlaufende Schwenkachse (54) gelagert sind, dass eine Wippenschwenkbewegung in einer durch die Griffhalbschalen (12, 14) aufgespannten Ebene liegt,
**dadurch gekennzeichnet, dass** zur Vorspannung der beiden Wippen jeweils eine Einstellfeder (36) sowie ein Gewindestift (34) zur Justierung vorgesehen sind und die beiden Wippen (30, 32) jeweils mindestens eine Schulter aufweisen, die als Anschlag für die Einstellfeder (36) und den Gewindestift (34) dient.

2. Betätigungselement (10) nach Anspruch 1, bei dem die Griffhalbschale (12, 14) einen der Einrastwippe (30, 32) zugeordneten Gewindestift (34) zur Feineinstellung der Wippenausrichtung aufweist.

3. Betätigungselement (10) zum Betätigen eines mit dem Griff in Wirkverbindung stehenden Instruments für minimalinvasive Eingriffe, mit
- zwei relativ zueinander bewegbaren Griffhalbschalen (12, 14), die einenends mittels eines Vorspannmechanismus (16, 18) miteinander verbunden sind, und
- einem im wesentlichen mittig zwischen den beiden Griffhalbschalen (12, 14) geführten Rohr (20), wobei zur Übertragung einer Betätigungsbewegung der Griffhalbschalen (12, 14) ein zwischen den Griffhalbschalen (12, 14) und dem Rohr (20) angeordneter Gelenkmechanismus (22, 24, 26) vorgesehen ist,
wobei das Betätigungselement (10) des weiteren einen Rastmechanismus (30, 32) aufweist, der eine an einer ersten Griffhalbschale (12) angeordnete Einrastwippe (30) und eine an einer zweiten Griffhalbschale (14) angeordnete Sperrwippe (32) umfasst, wobei die beiden Wippen (30, 32) bei zusammengeführten Griffhalbschalen (12, 14) durch einen dafür vorgesehenen Durchbruch in dem Rohr (20) in Sperrrastverbindung treten, und wobei die beiden Wippen (30, 32) derart um jeweils eine senkrecht zu einer Längsachse des Rohrs verlaufende Schwenkachse (54) gelagert sind, dass eine Wippenschwenkbewegung in einer durch die Griffhalbschalen (12, 14) aufgespannten Ebene liegt,
**dadurch gekennzeichnet, dass** zur Vorspannung der beiden Wippen jeweils eine Einstellfeder (36) sowie ein Gewindestift (76) zur Justierung vorgesehen sind und die beiden Wippen (30, 32) jeweils mindestens eine Schulter aufweisen, die als Anschlag für die Einstellfeder (36) dient, und die Sperrwippe (32) und/oder die Einrastwippe (30) jeweils eine Justierschraube (76) aufweisen.

4. Betätigungselement (10) zum Betätigen eines mit dem Griff in Wirkverbindung stehenden Instruments für minimalinvasive Eingriffe, mit
- zwei relativ zueinander bewegbaren Griffhalbschalen (12, 14), die einenends mittels eines Vorspannmechanismus (16, 18) miteinander verbunden sind, und
- einem im wesentlichen mittig zwischen den beiden Griffhalbschalen (12, 14) geführten Rohr (20), wobei zur Übertragung einer Betätigungsbewegung der Griffhalbschalen (12, 14) ein zwischen den Griffhalbschalen (12, 14) und dem Rohr (20) angeordneter Gelenkmechanismus (22, 24, 26) vorgesehen ist,
wobei das Betätigungselement (10) des weiteren einen Rastmechanismus (30, 32) aufweist, der eine an einer ersten Griffhalbschale (12) angeordnete Einrastwippe (30) und eine an einer zweiten Griffhalbschale (14) angeordnete Sperrwippe (32) umfasst, wobei die beiden Wippen (30, 32) bei zusammengeführten Griffhalbschalen (12, 14) durch einen dafür vorgesehenen Durchbruch in dem Rohr (20) in Sperrrastverbindung treten, und wobei die beiden Wippen (30, 32) derart um jeweils eine senkrecht zu einer Längsachse des Rohrs verlaufende Schwenkachse (54) gelagert sind, dass eine Wippenschwenkbewegung in einer durch die Griffhalbschalen (12, 14) aufgespannten Ebene liegt,
**dadurch gekennzeichnet, dass** zur Vorspannung der beiden Wippen jeweils eine Einstellfeder (36) vorgesehen ist und die beiden Wippen (30, 32) jeweils mindestens eine Schulter aufweisen, die als Anschlag für die Einstellfeder (36) dient, und die Griffhalbschale (12, 14) eine der Sperrwippe bzw. Einrastwippe (30, 32) zugeordnete Schulter (80) als Wippenanschlag aufweist.

5. Betätigungselement (10) nach einem der Ansprüche 1 bis 4, bei dem die beiden Wippen (30, 32) durch abermaliges Betätigen der zusammengeführten Griffhalbschalen (12, 14) aus der Sperrrastverbindung lösbar sind.

6. Betätigungselement (10) nach einem der Ansprüche 1 bis 5, dessen Sperrwippe (32) ein Rastelement (62) aufweist und dessen Einrastwippe (30) einen mit dem Rastelement (62) der Sperrwippe (32) in Wirkverbindung bringbaren Hinterschnitt (60) aufweist.

7. Betätigungselement (10) nach Anspruch 6, bei dem der Hinterschnitt (60) eine Bohrung aufweist.

8. Wippenpaar (30, 32) für ein Betätigungselement (10) zum Betätigen eines mit dem Griff in Wirkverbindung stehenden Instruments für minimalinvasive Eingriffe, umfassend eine Einrastwippe (30) und eine Sperrwippe (32), wobei die Sperrwippe (32) ein Rastelement (62) aufweist und die Einrastwippe (30) einen mit dem Rastelement (62) der Sperrwippe (32) in Wirkverbindung bringbaren Hinterschnitt (60) aufweist, **dadurch gekennzeichnet, dass** die beiden Wippen (30, 32) jeweils mindestens eine Schulter aufweisen, die als Anschlag für eine Einstellfeder (36) und einen Gewindestift (34) zur Justierung dient, die in dem Betätigungselement (10) zur Vorspannung der beiden Wippen vorgesehen sind.

9. Wippenpaar (30, 32) für ein Betätigungselement (10) zum Betätigen eines mit dem Griff in Wirkverbindung stehenden Instruments für minimalinvasive Eingriffe, umfassend eine Einrastwippe (30) und eine Sperrwippe (32), wobei die Sperrwippe (32) ein Rastelement (62) aufweist und die Einrastwippe (30) einen mit dem Rastelement (62) der Sperrwippe (32) in Wirkverbindung bringbaren Hinterschnitt (60) aufweist, **dadurch gekennzeichnet, dass** die beiden Wippen (30, 32) jeweils mindestens eine Schulter aufweisen, die als Anschlag für eine in dem Betätigungselement (10) zur Vorspannung der beiden Wippen vorgesehene Einstellfeder (36) dient, und die Sperrwippe (32) und/oder die Einrastwippe (30) jeweils eine Justierschraube (76) aufweisen.

10. Wippenpaar (30, 32) nach Anspruch 9, bei dem die Sperrwippe (32) und/oder die Einrastwippe (30) jeweils einen zur Aufnahme einer Stellschraube (76) ausgebildeten Ansatz (74) mit einer Gewindebohrung (70, 72) aufweisen.

11. Wippenpaar (30, 32) für ein Betätigungselement (10) zum Betätigen eines mit dem Griff in Wirkverbindung stehenden Instruments für minimalinvasive Eingriffe, umfassend eine Einrastwippe (30) und eine Sperrwippe (32), wobei die Sperrwippe (32) ein Rastelement (62) aufweist und die Einrastwippe (30) einen mit dem Rastelement (62) der Sperrwippe (32) in Wirkverbindung bringbaren Hinterschnitt (60) aufweist, **dadurch gekennzeichnet, dass** die beiden Wippen (30, 32) jeweils mindestens eine Schulter aufweisen, die als Anschlag für eine in dem Betätigungselement (10) zur Vorspannung der beiden Wippen vorgesehene Einstellfeder (36) und eine der Einrastwippe (30, 32) als Wippenanschlag zugeordnete Schulter (80) der Griffhalbschale (12, 14) dient.

12. Wippenpaar (30, 32) nach einem der Ansprüche 8 bis 11, bei dem die Sperrwippe (32) und die Einrastwippe (30) jeweils eine derart ausgebildete Schwenkachse (54) aufweisen, dass eine Wippenschwenkbewegung in einem in ein Betätigungselement (10) eingebauten Zustand im wesentlichen parallel zu einer Längserstreckung des Betätigungselements (10) erfolgt.

## Claims

1. Actuating element (10) for actuating an instrument, operatively connected to the handle, for minimally invasive operations, having
- two handle half-shells (12, 14) that are movable relative to one another and are connected together at one end by means of a preloading mechanism (16, 18), and
- a tube (20) guided substantially centrally between the two handle half-shells (12, 14), wherein, in order to transmit an actuating movement of the handle half-shells (12, 14), a joint mechanism (22, 24, 26) arranged between the handle half-shells (12, 14) and the tube (20) is provided, wherein the actuating element (10) furthermore has a latch mechanism (30, 32), which comprises a latching rocker (30) arranged on a first handle half-shell (12), and a locking rocker (32) arranged on a second handle half-shell (14), wherein, with the handle half-shells (12, 14) moved together, the two rockers (30, 32) pass into locking-latching connection through an aperture, provided for this purpose, in the tube (20), and wherein the two rockers (30, 32) are mounted about a respective pivot pin (54), extending perpendicularly to a longitudinal axis of the tube, such that a rocker pivoting movement lies in a plane defined by the handle half-shells (12, 14), **characterized in that**, in order to preload the two rockers, a respective adjusting spring (36) and a threaded pin (34) for adjustment are provided and the two rockers (30, 32) each have at least one shoulder, which serves as an abutment for the adjusting spring (36) and the threaded pin (34).

2. Actuating element (10) according to Claim 1, in which the handle half-shell (12, 14) has a threaded pin (34), assigned to the latching rocker (30, 32), for the fine adjustment of the rocker orientation.

3. Actuating element (10) for actuating an instrument, operatively connected to the handle, for minimally invasive operations, having
- two handle half-shells (12, 14) that are movable relative to one another and are connected together at one end by means of a preloading mechanism (16, 18), and
- a tube (20) guided substantially centrally between the two handle half-shells (12, 14), wherein, in order to transmit an actuating movement of the handle half-shells (12, 14), a joint mechanism (22, 24, 26) arranged between the handle half-shells (12, 14) and the tube (20) is provided, wherein the actuating element (10) furthermore has a latch mechanism (30, 32), which comprises a latching rocker (30) arranged on a first handle half-shell (12), and a locking rocker (32) arranged on a second handle half-shell (14), wherein, with the handle half-shells (12, 14) moved together, the two rockers (30, 32) pass into locking-latching connection through an aperture, provided for this purpose, in the tube (20), and wherein the two rockers (30, 32) are mounted about a respective pivot pin (54), extending perpendicularly to a longitudinal axis of the tube, such that a rocker pivoting movement lies in a plane defined by the handle half-shells (12, 14), **characterized in that**, in order to preload the two rockers, a respective adjusting spring (36) and a threaded pin (76) for adjustment are provided and the two rockers (30, 32) each have at least one shoulder, which serves as an abutment for the adjusting spring (36), and the locking rocker (32) and/or the latching rocker (30) each have an adjusting screw (76).

4. Actuating element (10) for actuating an instrument, operatively connected to the handle, for minimally invasive operations, having
- two handle half-shells (12, 14) that are movable relative to one another and are connected together at one end by means of a preloading mechanism (16, 18), and
- a tube (20) guided substantially centrally between the two handle half-shells (12, 14), wherein, in order to transmit an actuating movement of the handle half-shells (12, 14), a joint mechanism (22, 24, 26) arranged between the handle half-shells (12, 14) and the tube (20) is provided, wherein the actuating element (10) furthermore has a latch mechanism (30, 32), which comprises a latching rocker (30) arranged on a first handle half-shell (12), and a locking rocker (32) arranged on a second handle half-shell (14), wherein, with the handle half-shells (12, 14) moved together, the two rockers (30, 32) pass into locking-latching connection through an aperture, provided for this purpose, in the tube (20), and wherein the two rockers (30, 32) are mounted about a respective pivot pin (54), extending perpendicularly to a longitudinal axis of the tube, such that a rocker pivoting movement lies in a plane defined by the handle half-shells (12, 14), **characterized in that**, in order to preload the two rockers, a respective adjusting spring (36) is provided and the two rockers (30, 32) each have at least one shoulder, which serves as an abutment for the adjusting spring (36), and the handle half-shell (12, 14) has a shoulder (80), assigned to the locking rocker or latching rocker (30, 32), as rocker abutment.

5. Actuating element (10) according to one of Claims 1 to 4, in which the two rockers (30, 32) are releasable from the locking-latching connection by the moved-together handle half-shells (12, 14) being actuated again.

6. Actuating element (10) according to one of Claims 1 to 5, the locking rocker (32) of which has a latch element (62) and the latching rocker (30) of which has an undercut (60) that is able to be operatively connected to the latch element (62) of the locking rocker (32).

7. Actuating element (10) according to Claim 6, in which the undercut (60) has a bore.

8. Rocker pair (30, 32) for an actuating element (10) for actuating an instrument, operatively connected to the handle, for minimally invasive operations, comprising a latching rocker (30) and a locking rocker (32), wherein the locking rocker (32) has a latch element (62) and the latching rocker (30) has an undercut (60) that is able to be operatively connected to the latch element (62) of the locking rocker (32), **characterized in that** the two rockers (30, 32) each have at least one shoulder, which serves as an abutment for an adjusting spring (36) and a threaded pin (34) for adjustment, which are provided in the actuating element (10) for preloading the two rockers.

9. Rocker pair (30, 32) for an actuating element (10) for actuating an instrument, operatively connected to the handle, for minimally invasive operations, comprising a latching rocker (30) and a locking rocker (32), wherein the locking rocker (32) has a latch element (62) and the latching rocker (30) has an undercut (60) that is able to be operatively connected to the latch element (62) of the locking rocker (32), **characterized in that** the two rockers (30, 32) each have at least one shoulder, which serves as an abutment for an adjusting spring (36) provided in the actuating element (10) for preloading the two rockers, and the locking rocker (32) and/or the latching rocker (30) each have an adjusting screw (76).

10. Rocker pair (30, 32) according to Claim 9, in which the locking rocker (32) and/or the latching rocker (30) each have a projection (74), configured to receive a setscrew (76), with a threaded bore (70, 72).

11. Rocker pair (30, 32) for an actuating element (10) for actuating an instrument, operatively connected to the handle, for minimally invasive operations, comprising a latching rocker (30) and a locking rocker (32), wherein the locking rocker (32) has a latch element (62) and the latching rocker (30) has an undercut (60) that is able to be operatively connected to the latch element (62) of the locking rocker (32), **characterized in that** the two rockers (30, 32) each have at least one shoulder, which serves as an abutment for an adjusting spring (36) provided in the actuating element (10) for preloading the two rockers and a shoulder (80), assigned to the latching rocker (30, 32) as rocker abutment, of the handle half-shell (12, 14).

12. Rocker pair (30, 32) according to one of Claims 8 to 11, in which the locking rocker (32) and the latching rocker (30) each have a pivot pin (54) configured such that a rocker pivoting movement takes place, in a state installed in an actuating element (10), substantially parallel to a longitudinal extent of the actuating element (10).

## Revendications

1. Elément d'actionnement (10) pour l'actionnement d'un instrument pour interventions minimalement invasives, qui est en liaison active avec la poignée, comportant
- deux demi-coques de poignée (12, 14) qui sont mobiles l'une par rapport à l'autre et qui sont reliées entre elles à une extrémité au moyen d'un mécanisme de précontrainte (16, 18), et
- un tube (20) guidé de manière sensiblement centrale entre les deux demi-coques de poignée (12, 14), un mécanisme d'articulation (22, 24, 26) disposé entre les demi-coques de poignée (12, 14) et le tube (20) étant prévu pour transmettre un mouvement d'actionnement des demi-coques de poignée (12, 14), dans lequel
l'élément d'actionnement (10) comprend en outre un mécanisme d'enclenchement (30, 32) qui comprend une bascule d'enclenchement (30) disposée sur une première demi-coque de poignée (12) et une bascule de blocage (32) disposée sur une seconde demi-coque de poignée (14), et
lorsque les demi-coques de poignée (12, 14) sont réunies, les deux bascules (30, 32) viennent en liaison d'enclenchement et de blocage à travers une traversée prévue à cet effet dans le tube (20), et
les deux bascules (30, 32) sont montées autour d'un axe de pivotement (54) perpendiculaire à un axe longitudinal du tube, de telle sorte qu'un mouvement de pivotement de la bascule se déroule dans un plan défini par les demi-coques de poignée (12, 14),
**caractérisé en ce que**
pour précontraindre les deux bascules, il est prévu un ressort de réglage respectif (36), et pour les ajuster, il est prévu un goujon fileté respectif (34), les deux bascules (30, 32) présentant chacune au moins un épaulement qui sert de butée pour le ressort de réglage (36) et pour le goujon fileté (34).

2. Elément d'actionnement (10) selon la revendication 1,
dans lequel
la demi-coque de poignée (12, 14) comprend un goujon fileté (34) associé à la bascule d'enclenchement (30, 32) pour l'ajustement fin de l'orientation de la bascule.

3. Elément d'actionnement (10) pour l'actionnement d'un instrument pour interventions minimalement invasives, qui est en liaison active avec la poignée, comportant
- deux demi-coques de poignée (12, 14) qui sont mobiles l'une par rapport à l'autre et qui sont reliées entre elles à une extrémité au moyen d'un mécanisme de précontrainte (16, 18), et
- un tube (20) guidé de manière sensiblement centrale entre les deux demi-coques de poignée (12, 14), un mécanisme d'articulation (22, 24, 26) disposé entre les demi-coques de poignée (12, 14) et le tube (20) étant prévu pour transmettre un mouvement d'actionnement des demi-coques de poignée (12, 14), dans lequel
l'élément d'actionnement (10) comprend en outre un mécanisme d'enclenchement (30, 32) qui comprend une bascule d'enclenchement (30) disposée sur une première demi-coque de poignée (12) et une bascule de blocage (32) disposée sur une seconde demi-coque de poignée (14), et
lorsque les demi-coques de poignée (12, 14) sont réunies, les deux bascules (30, 32) viennent en liaison d'enclenchement et de blocage à travers une traversée prévue à cet effet dans le tube (20), et
les deux bascules (30, 32) sont montées autour d'un axe de pivotement (54) perpendiculaire à un axe longitudinal du tube, de telle sorte qu'un mouvement de pivotement de la bascule se déroule dans un plan défini par les demi-coques de poignée (12, 14),
**caractérisé en ce que**
pour précontraindre les deux bascules, il est prévu un ressort de réglage respectif (36), et pour les ajuster, il est prévu un goujon fileté respectif (76), les deux bascules (30, 32) présentant chacune au moins un épaulement qui sert de butée pour le ressort de réglage (36), et la bascule de blocage (32) et/ou la bascule d'enclenchement (30) comprennent chacune une vis d'ajustement (76).

4. Elément d'actionnement (10) pour l'actionnement d'un instrument pour interventions minimalement invasives, qui est en liaison active avec la poignée, comportant
- deux demi-coques de poignée (12, 14) qui sont mobiles l'une par rapport à l'autre et qui sont reliées entre elles à une extrémité au moyen d'un mécanisme de précontrainte (16, 18), et
- un tube (20) guidé de manière sensiblement centrale entre les deux demi-coques de poignée (12, 14), un mécanisme d'articulation (22, 24, 26) disposé entre les demi-coques de poignée (12, 14) et le tube (20) étant prévu pour transmettre un mouvement d'actionnement des demi-coques de poignée (12, 14), dans lequel
l'élément d'actionnement (10) comprend en outre un mécanisme d'enclenchement (30, 32) qui comprend une bascule d'enclenchement (30) disposée sur une première demi-coque de poignée (12) et une bascule de blocage (32) disposée sur une seconde demi-coque de poignée (14), et
lorsque les demi-coques de poignée (12, 14) sont réunies, les deux bascules (30, 32) viennent en liaison d'enclenchement et de blocage à travers une traversée prévue à cet effet dans le tube (20), et
les deux bascules (30, 32) sont montées autour d'un axe de pivotement (54) perpendiculaire à un axe longitudinal du tube, de telle sorte qu'un mouvement de pivotement de la bascule se déroule dans un plan défini par les demi-coques de poignée (12, 14),
**caractérisé en ce que**
pour précontraindre les deux bascules, il est prévu un ressort de réglage respectif (36), et les deux bascules (30, 32) présentent chacune au moins un épaulement qui sert de butée pour le ressort de réglage (36), et la demi-coque de poignée (12, 14) comprend un épaulement (80) à titre de butée, qui est associé à la bascule de blocage ou à la bascule d'enclenchement (30, 32) .

5. Elément d'actionnement (10) selon l'une des revendications 1 à 4,
dans lequel
les deux bascules (30, 32) peuvent être détachées de la liaison par blocage et par enclenchement par une répétition de l'actionnement des demi-coques de poignée (12, 14) réunies.

6. Elément d'actionnement (10) selon l'une des revendications 1 à 5,
dont la bascule de blocage (32) comprend un élément d'enclenchement et dont la bascule d'enclenchement (30) comprend une contre-dépouille (60) qui peut être amenée en liaison d'action avec l'élément d'enclenchement (62) de la bascule de blocage (32) .

7. Elément d'actionnement (10) selon la revendication 6,
dans lequel
la contre-dépouille (60) présente un perçage.

8. Paire de bascules (30, 32) pour un élément d'actionnement (10) destiné à actionner un instrument pour interventions minimalement invasives, qui est en liaison d'action avec la poignée, comprenant une bascule d'enclenchement (30) et une bascule de blocage (32), la bascule de blocage (32) présentant un élément d'enclenchement (62), et la bascule d'enclenchement (30) présentant une contre-dépouille (60) qui peut être mise en liaison active avec l'élément d'enclenchement (62) de la bascule de blocage (32),
**caractérisée en ce que**
les deux bascules (30, 32) présentent chacune au moins un épaulement qui sert de butée pour un ressort de réglage (36) et/ou pour un goujon fileté (34) pour l'ajustement, qui sont prévus dans l'élément d'actionnement (10) pour précontraindre les deux bascules.

9. Paire de bascules (30, 32) pour un élément d'actionnement (10) destiné à actionner un instrument pour interventions minimalement invasives, qui est en liaison d'action avec la poignée, comprenant une bascule d'enclenchement (30) et une bascule de blocage (32), la bascule de blocage (32) présentant un élément d'enclenchement (62), et la bascule d'enclenchement (30) présentant une contre-dépouille (60) qui peut être mise en liaison active avec l'élément d'enclenchement (62) de la bascule de blocage (32),
**caractérisée en ce que**
les deux bascules (30, 32) présentent chacune au moins un épaulement qui sert de butée pour un ressort de réglage (36) prévu dans l'élément d'actionnement (10) pour précontraindre les deux bascules, et la bascule de blocage (32) et/ou la bascule d'enclenchement (30) comprennent chacune une vis d'ajustement (76).

10. Paire de bascules (30, 32) selon la revendication 9,
dans laquelle
la bascule de blocage (32) et/ou la bascule d'enclenchement (30) présentent chacune un talon (74) pourvu d'un perçage taraudé (70, 72) et réalisé pour recevoir une vis de réglage (76) .

11. Paire de bascules (30, 32) pour un élément d'actionnement (10) destiné à actionner un instrument pour interventions minimalement invasives, qui est en liaison d'action avec la poignée, comprenant une bascule d'enclenchement (30) et une bascule de blocage (32), la bascule de blocage (32) présentant un élément d'enclenchement (62), et la bascule d'enclenchement (30) présentant une contre-dépouille (60) qui peut être mise en liaison active avec l'élément d'enclenchement (62) de la bascule de blocage (32),
**caractérisée en ce que**
les deux bascules (30, 32) présentent chacune au moins un épaulement qui sert de butée pour un ressort de réglage (36) prévu dans l'élément d'actionnement (10) pour précontraindre les deux bascules, et un épaulement (80) de la demi-coque de poignée (12, 14) associé en tant que butée à la bascule d'enclenchement (30, 32).

12. Paire de bascules (30, 32) selon l'une des revendications 8 à 11,
dans laquelle
la bascule de blocage (32) et la bascule d'enclenchement (30) présentent chacune un axe de pivotement (54) réalisé de telle sorte que dans un état monté dans un élément d'actionnement, un mouvement de pivotement de la bascule se déroule sensiblement parallèlement à une extension longitudinale de l'élément d'actionnement (10).
